# EUROPÄISCHE PATENTSCHRIFT

(11) **EP 2 114 276 B1**
(45) Veröffentlichungstag und Bekanntmachung des Hinweises auf die Patenterteilung: **16.05.2012**
(21) Anmeldenummer: 07856570.2
(22) Anmeldetag: 11.12.2007
(51) Int. Cl.: A61B 18/02

(54) **KRYOCHIRURGISCHES INSTRUMENT ZUM ABTRENNEN EINER GEWEBEPROBE VON UMLIEGENDEM GEWEBE EINES ZU BEHANDELNDEN BIOLOGISCHEN GEWEBES**
CRYOSURGICAL INSTRUMENT FOR SEPARATING A TISSUE SAMPLE FROM SURROUNDING TISSUE OF A BIOLOGICAL TISSUE THAT IS TO BE TREATED
INSTRUMENT CRYOCHIRURGICAL POUR SÉPARER UN ÉCHANTILLON DE TISSU BIOLOGIQUE À TRAITER DU TISSU ENVIRONNANT

(30) Priorität: 19.12.2006 DE 102006059999; 02.05.2007 DE 102007020582
(43) Veröffentlichungstag der Anmeldung: 11.11.2009
(73) Patentinhaber: ERBE Elektromedizin GmbH, 72072 Tübingen (DE)
(72) Erfinder: SIGLE, Irina, 72116 Mössingen (DE); VOIGTLÄNDER, Mathias, 72810 Gomaringen (DE); SCHÄLLER, Daniel, 72074 Tübingen (DE); SZYRACH, Mara, 72070 Tübingen (DE); FISCHER, Klaus, 72202 Nagold (DE)
(74) Vertreter: Bohnenberger, Johannes
(86) Internationale Anmeldenummer: PCT/EP2007/010822
(87) Internationale Veröffentlichungsnummer: WO 2008/074422

(56) Entgegenhaltungen:
- WO-A-01/67975
- WO-A-01/97702
- US-A- 6 032 675
- US-A1- 2003 181 896

## Beschreibung

Die Erfindung betrifft ein kryochirurgisches Instrument nach dem Oberbegriff des Patentanspruches 1 .

In der Kryochirurgie wird die gezielte, kontrollierte Kälteanwendung zur Devitalisierung von biologischem Gewebe eingesetzt. Insbesondere mit flexiblen Sonden werden auch Fremdkörper aus Körperhöhlen durch Festfrieren an der Kryosonde bzw. an einem Sondenkopf extrahiert, so z.B. verschluckte und dabei versehentlich eingeatmete Fremdkörper, die aus den Atemwegen entfernt werden müssen. Die Kryochirurgie eignet sich aber auch zur Gewinnung von Gewebeproben (Biopsie). Dabei friert ein bestimmter Gewebebereich, die Gewebeprobe, an den Sondenkopf an, und kann nach dem Abtrennen von umliegendem Gewebe einer Untersuchung zugänglich gemacht werden.

Um in der Chirurgie tiefzufrieren, gibt es verschiedene Möglichkeiten. Eine stützt sich auf den Joule-Thomson-Effekt: die Atome bzw. Moleküle eines sich expandierenden Gases unterhalb der Inversionstemperatur arbeiten gegen die gegenseitige Anziehung an, so dass das Gas an innerer Energie verliert. Es kühlt ab. Als expandierendes Gas - im Folgenden Arbeitsgas oder Kühlgas genannt - werden üblicherweise CO₂ oder N₂O eingesetzt.

Kryochirurgische Instrumente der eben beschriebenen Art verfügen üblicherweise über eine Sonde, die an das zu behandelnde Gewebe verbracht werden kann, ferner über Gasleitungseinrichtungen, welche die Sonden durchsetzen und innerhalb der Sonden das Arbeitsgas in das Innenlumen der Sonden entlassen, wo es expandiert und in Folge dessen die Spitzen der Sonde, einen Sondenkopf, abkühlt. Da der Sondenkopf vorzugsweise aus thermisch leitfähigem Material gefertigt ist, ist ein Ableiten der Gewebewärme über den Sondenkopf und damit eine Kühlwirkung gewährleistet.

Auf herkömmlichem Wege werden Gewebeproben meist mittels Zangenbiopsie gewonnen. Das gewonnene Präparat ist jedoch sehr klein und wird bei der Entnahme meist gequetscht. Die Biopsie mittels Kryochirurgie ermöglicht eine wesentlich effizientere Probengewinnung. Zum Zwecke einer Biopsie wird die Kryosonde (starr oder flexibel) üblicherweise über einen Arbeitskanal eines Endoskops (ebenfalls starr oder flexibel) an die gewünschte Stelle, z. B. im Gastrointestinaltrakt, herangeführt. Die Sondenspitze, d. h. der Sondenkopf wird auf das zu behandelnden Gewebe, z. B. die Schleimhaut, aufgesetzt und ein Gewebebereich, die Gewebeprobe, friert am Sondenkopf aufgrund oben beschriebener Kühlmechanismen fest. Das Gewebe bzw. die spätere Gewebeprobe haftet somit an dem gekühlten Sondenkopf, und durch eine kurze Zugbewegung wird das gefrorene Gewebe aus dem umliegenden Gewebe herausgelöst.

Das Ablösen bedarf eines relativ großen Kraftaufwandes, der vom Anwender aufgebracht werden muss. Dies ist insbesondere dann problematisch, wenn sich das zu behandelnde Gewebe während des Abtrennvorganges (also bei einer Zugbewegung) mitbewegt. So kann z. B. auf den Dickdarm (wenn von diesem ein Biopsat gewonnen werden soll) keine große Zugkraft ausgeübt werden, da dieser schwimmend im Abdomen liegt. Die notwendige Zugkraft kann hier - wenn überhaupt - nur in Impulsen aufgebracht werden. Dabei können Verletzungen im umliegenden Gewebe auftreten oder eine Gewebeprobe lässt sich überhaupt nicht entnehmen, weil das umliegende Gewebe zu sehr nachgibt. Zum Beispiel wird unter Umständen das festgefrorene Gewebe durch die Zugkraft von dem Sondenkopf vorzeitig gelöst.

Dokument WO01/97702 zeigt ein Instrument nach dem Oberbegriff des Patentanspruchs 1.

Der Erfindung liegt daher die Aufgabe zugrunde, ein kryochirurgisches Instrument der eingangs genannten Art dahin gehend weiter zu bilden, dass eine Gewebeprobe zuverlässig und gewebeschonend entnommen werden kann, wobei ein hohes Maß an Sicherheit für den Patienten gegeben sein soll.

Diese Aufgabe wird durch ein kryochirurgisches Instrument nach Patentanspruch 1 gelöst.

Insbesondere wird die Aufgabe vorrichtungsmäßig durch ein kryochirurgisches Instrument gelöst, das eine Sonde zum Heranführen eines Sondenkopfes an ein zu behandelndes biologisches Gewebe umfasst, sowie Gasleitungseinrichtungen zum Zuführen von Kühlgas von einer Gasquelle an den Sondenkopf und zum Abführen des Kühlgases von diesem, wobei der Sondenkopf derart ausgebildet ist, dass mittels des zugeführten Gases ein begrenzter Gewebebereich zur Gewinnung einer Gewebeprobe kühlbar und von umliegendem Gewebe in einem an dem Sondenkopf angefrorenen Zustand abtrennbar ist. Das Instrument weist eine Stützeinrichtung auf, in der die Sonde geführt und die relativ zu der Sonde derart bewegbar ist, dass mittels der Stützeinrichtung das umliegende Gewebe während des Abtrennens der Gewebeprobe abstützbar ist.

Ein wesentlicher Punkt der Erfindung liegt darin, dass mittels der Stützeinrichtung ein Gegenlager geschaffen ist, das während des Abtrennens der Gewebeprobe von dem umliegenden Gewebe dieses stützt und in Position hält. Sonde und Stützeinrichtung sind dazu derart relativ zueinander bewegbar, dass die Stützeinrichtung auf dem die Gewebeprobe umgebenden Gewebe zum Aufliegen kommt, während über die Sonde am Gewebe (im Prinzip am Biopsat) gezogen wird. Das heißt, Stützeinrichtung und Sonde sind derart zueinander bewegbar, dass entweder der Sondenkopf und ggf. Teile eines Sondenkörpers der Sonde freigelegt oder die Stützeinrichtung über den Sondenkopf hinausbewegt werden kann. Durch das Aufsetzen der Stützeinrichtung auf das Gewebe wirken eine Kraft der Stützeinrichtung am umgebenden Gewebe und gegengleich eine Zugkraft durch den Zug am gefrorenen Gewebe über die Sonde (Actio gegengleich Reactio), wenn sich das gefrorene Gewebe mittels des Sondenkopfes von dem umgebenden Gewebe entfernt. Die Stützeinrichtung übt also eine Gegenkraft auf das Gewebe aus, während über die Sonde an dem am Sondenkopf angefrorenen Gewebeteil gezogen wird. Die Zugkraft wirkt damit nur auf einen kleinen Flächenbereich und nicht auf das gesamte umliegende Gewebe. Damit kann das umliegende Gewebe im Wesentlichen in seiner ursprünglichen Position belassen werden und wird nicht ungünstig beansprucht.

In einer bevorzugten Ausführungsform weist die Sonde einen starren oder flexiblen Schaft oder Katheter auf und ist durch einen Instrumentenkanal eines starren oder flexiblen Endoskops an das zu behandelnde Gewebe heranführbar. Das heißt, die Sonde mit Sondenkörper und Sondenkopf ist vorzugsweise für endoskopische Eingriffe ausgebildet und kann so auf einfachem Wege an ein Operationsgebiet herangeführt werden.

Vorzugsweise weist das Instrument eine Griffeinrichtung zur Handhabung der Sonde auf, wobei ein proximales Ende der Sonde in der Griffeinrichtung gelagert ist. Die Griffeinrichtung erleichtert die Handhabung des Instruments.

Vorzugsweise umfassen die Gasleitungseinrichtungen eine durch die Sonde verlaufende Gaszuführleitung, so dass ein Hohlraum im Inneren der Sonde bzw. des Sondenkörpers zum Kühlen des Sondenkopfes mit Gas befüllbar ist. Da der Sondenkopf mittels des Arbeitsgases bzw. Kühlgases gekühlt werden muss, sind die Gasleitungseinrichtungen derart ausgebildet, dass das Kühlgas mit dem Sondenkopf in Kontakt bringbar ist. Hierzu ist beispielsweise in unmittelbarer Nähe des Sondenkopfes ein Hohlraum angeordnet, der mit dem Kühlgas befüllbar ist. Das Gas strömt also durch die Gaszuführleitung innerhalb der Sonde in Richtung Sondenkopf in den Hohlraum und kühlt dabei (z. B. aufgrund der Entspannung, wie oben beschrieben) den Sondenkopf. Die Gaszuführleitung ist vorzugsweise derart angeordnet, dass sie innerhalb einer ebenfalls in der Sonde angeordneten Gasabführleitung der Gasleitungseinrichtungen liegt, wobei die Gasabführleitung vorzugsweise derart ausgebildet ist, dass sie den Hohlraum umfasst.

Die Gasleitungseinrichtungen umfassen nicht nur die Leitungsabschnitte (Gaszuführleitung, Gasabführleitung) innerhalb der Sonde, sondern führen in einer Ausführungsform über die Griffeinrichtung durch eine Schlaucheinrichtung. Die Schlaucheinrichtung lässt sich dann über eine weitere Verlängerung der Gasleitungseinrichtungen oder unmittelbar an das kryochirurgische Gerät anschließen. Das kryochirurgische Instrument umfasst also vorzugsweise die Sonde mit der Griffeinrichtung und der Schlaucheinrichtung zum Anschließen an das kryochirurgische Gerät.

Kryochirurgiegeräte sind für verschiedenste Anwendungszwecke vorgesehen und arbeiten beispielsweise nach dem oben erwähnten Joule-Thomson-Effekt. Möglich wäre es auch, kryochirurgische Eingriffe mittels flüssigem Stickstoff durchzuführen.

Vorzugsweise ist die Stützeinrichtung derart ausgebildet, dass sie die Sonde rohr- oder schlauchförmig umschließt. Das heißt, die Stützeinrichtung ist als ein Schlauch oder ein Rohr ausgebildet, in welches die Sonde eingeführt ist. Das Stützrohr ist hier also als ein äußerer Sondenkörper vorgesehen. Stützeinrichtung und Sonde sind derart zueinander angeordnet, dass sie relativ zueinander oder auch gegeneinander bewegbar sind. Durch das gegeneinander Bewegen lässt sich der Sondenkopf über ein sondenkopfnahes (distales) Ende der Stützeinrichtung hinaus bewegen und liegt somit frei (so dass der Sondenkopf zur Entnahme der Gewebeprobe das Gewebe erreichen kann). Umgekehrt lässt sich das Stützrohr über den Sondenkopf hinaus schieben und der Sondenkopf ist in das Stützrohr (mit der Gewebeprobe) vollständig aufnehmbar. Damit kann das Stützrohr gegen das Gewebe, aus welchem die Gewebeprobe entnommen werden soll, zu dessen Abstützung geschoben werden, nachdem die Gewebeprobe an dem Sondenkopf aufgrund des Einfrierens angelagert ist.

In einer Ausführungsform ist eine Aufnahmeeinrichtung zur Aufnahme eines proximalen Endes der Stützeinrichtung vorgesehen, wobei die Aufnahmeeinrichtung und die Griffeinrichtung verschieblich zueinander mittels einer Kopplungseinheit miteinander verbunden sind. Dies ermöglicht schließlich das gegeneinander Bewegen von Sonde und Stützeinrichtung, indem Aufnahmeeinrichtung und Griffeinrichtung gegeneinander bewegt werden. Aufnahmeeinrichtung und Griffeinrichtung (und damit Stützeinrichtung und Sonde) sind über eine definierte Weglänge gegeneinander verschiebbar, wobei sich die Weglänge z. B. über entsprechend zusammenwirkende Anschläge zwischen Aufnahmeeinrichtung und Griffeinrichtung, also in der Kopplungseinheit, festlegen lässt.

Wie bereits oben erläutert, ist die Sonde vorzugsweise in der Stützeinrichtung geführt. Sonde und Stützeinrichtung sind also z. B. koaxial zueinander angeordnet, wobei die Stützeinrichtung die Sonde nur bis zur Aufnahmeeinrichtung umschließt, während die Sonde durch die Aufnahmeeinrichtung hindurch in die Griffeinrichtung hinein weiter verlaufen kann.

Vorzugsweise ist die Kopplungseinheit derart ausgebildet, dass eine Schubeinrichtung der Aufnahmeeinrichtung und ein Kanalbereich der Griffeinrichtung mindestens in Teilbereichen ineinander greifen, so dass die Stützeinrichtung und die Sonde mittels einer Schub- oder Zugbewegung von Griffeinrichtung und/oder Aufnahmeeinrichtung gegeneinander längs einer Erstreckungsrichtung der Sonde über die definierte Weglänge derart bewegbar sind, dass mindestens der Sondenkopf in die Stützeinrichtung aufnehmbar oder von dieser freigebbar ist. Die z. B. rohrförmige Schubeinrichtung ist also mit einem Ende in dem Kanalbereich der Griffeinrichtung geführt, so dass die Schubeinrichtung und die Griffeinrichtung voneinander weg und zueinander hin bewegt werden können. Das heißt, dass eine Relativbewegung zwischen Sonde und Stützeinrichtung derart erfolgt, dass die an der Griffeinrichtung befestigte Sonde (zumindest mit einem distalen Ende) aus der an der Aufnahmeeinrichtung befestigten Stützeinrichtung heraus bewegt werden kann und umgekehrt, die Stützeinrichtung derart weit über den Sondenkopf schiebbar ist, dass sie das umliegende Gewebe zurückhält, während die Gewebeprobe abgetrennt wird. Die Schubeinrichtung ist hier vorzugsweise deshalb rohrförmig ausgebildet, damit die Sonde bzw. der Sondenkörper weiter bis zur Griffeinrichtung bzw. in diese hinein geführt werden kann.

Je nach Ausmaß von Stützeinrichtung und Arbeitskanal des Endoskops kann die Stützeinrichtung derart fest in dem Arbeitskanal angeordnet (im Prinzip eingeklemmt) sein, dass eine Relativbewegung zwischen Stützeinrichtung und Arbeitskanal nur schwer möglich ist. Hier würde dann im Wesentlichen die Sonde über die Griffeinrichtung bewegt und in dem Stützrohr verschoben werden. Auch kann darauf abgestellt werden, die Stützeinrichtung über die Schubeinrichtung zu bewegen. In jedem Falle ist die Anordnung derart vorgesehen, dass es auf die Relativbewegung zwischen der Sonde an der Griffeinrichtung und der Stützeinrichtung an der Aufnahmeeinrichtung ankommt, wobei Sonde und Stützeinrichtung auch beide gegeneinander bewegbar sein können; die Stützeinrichtung muss während des Abtrennens der Gewebeprobe derart weit über den Sondenkopf hinausschiebbar bzw. der Sondenkopf in die Stützeinrichtung aufnehmbar sein, dass eine Gewebeabstützung und ein Abtrennen durchführbar sind. Die verschiebliche Lagerung der Aufnahmeeinrichtung in der Griffeinrichtung ermöglicht die Relativbewegung.

Vorzugsweise ist die Stützeinrichtung derart ausgebildet, dass der an den Sondenkopf angefrorene Gewebebereich (also die Gewebeprobe) mit dem Sondenkopf in die Stützeinrichtung aufnehmbar ist. Das heißt, um das Biopsat zu dessen Bergung in das Lumen der Stützeinrichtung, also z. B. in das Lumen des Stützrohres aufnehmen zu können, muss die Stützeinrichtung ein hinreichend großes Lumen aufweisen. Allerdings ist die die Sonde aufweisende Stützeinrichtung derart auszubilden, dass sie unproblematisch in einem Arbeitskanal eines Endoskops eingeführt werden kann. Die Möglichkeit, die Gewebeprobe in die Stützeinrichtung aufnehmen zu können, erleichtert das Bergen des Biopsats. Die Sonde kann aus dem Arbeitskanal herausgezogen werden, wobei das Biopsat sicher in dem Lumen der Gewebeprobe untergebracht ist.

Ist die Stützeinrichtung als ein Rohr ausgebildet, so ist das Gewebe über eine Öffnung der Stützeinrichtung umschließende Aufsetzkante an dem distalen Ende der Stützeinrichtung kontaktierbar. Diese Aufsetzkante ist bevorzugt abgestumpft ausgebildet, damit diese nicht in das Gewebe einschneidet. Allerdings muss bei der Ausbildung der Aufsetzkante darauf geachtet werden, dass diese durch den Arbeitskanal des Endoskops hindurch geführt werden kann. Ferner kann für die Stützeinrichtung ein Material verwendet werden, das sich bis zu einem gewissen Grad elastisch verformen lässt. Auch damit lässt sich eine Verletzung des umliegenden Gewebes vermeiden.

Der vorzugsweise aus Metall (z. B. Edelstahl) ausgebildete Sondenkopf ist mit einer vorzugsweise aus Kunststoff ausgebildeten Sonde verbunden. Als Sondenmaterial (Material für den Sondenkörper) ist beispielsweise ein Polyetherketon (PEK), z. B. ein Polyetheretherketon (PEEK) vorgesehen. PEEK besitzt eine hohe Festigkeit, eine gute Chemikalienbeständigkeit und eine sehr gute Temperaturfestigkeit. Die Stützeinrichtung ist vorzugsweise aus Perfluor-(Ethylen-Propylen)-Kunststoff (FEP), aus Polytetrafluorethylen (PTFE) oder dergleichen Kunststoff ausgebildet.

Weiterhin ist ein Verfahren zum Abtrennen einer Gewebeprobe von umliegendem Gewebe eines zu behandelnden biologischen Gewebes mit einem kryochirurgischen Instrument offenbart, das nicht Teil der Erfindung ist, das eine Sonde mit Sondenkopf aufweist sowie Gasleitungseinrichtungen für Kühlgas von einer Gasquelle eines kryochirurgischen Gerätes mit einer jeweils durch die Sonde verlaufenden Gaszuführleitung und Gasabführleitung hin zum Sondenkopf und weg von diesem und eine Stützeinrichtung, in der die Sonde geführt ist und die relativ zu der Sonde bewegbar ist, folgende Schritte vorgesehen sind:
- Heranführen der Sonde an das zu behandelnde Gewebe, vorzugsweise über einen Arbeitskanal eines Endoskops,
- Aufsetzen des Sondenkopfes auf das zu behandelnde Gewebe,
- Zuführen von Kühlgas zur Kühlung des Sondenkopfes, so dass ein begrenzter Gewebebereich zur Gewinnung der Gewebeprobe gekühlt wird und an den Sondenkopf anfriert,
- gegeneinander Bewegen von Stützeinrichtung und Sonde derart, dass die Gewebeprobe abgetrennt und das umliegende Gewebe mittels der Stützeinrichtung während des Abtrennens der Gewebeprobe abgestützt wird,
- Bergen der Gewebeprobe.

Mittels dieses Verfahrens lässt sich zuverlässig und mit einem hohen Maß an Sicherheit für den Patienten eine Gewebeprobe entnehmen, da die Stützeinrichtung das Gewebe, von welchem die Gewebeprobe entnommen werden soll, auch dann in der entsprechenden Position hält, wenn eine Zugkraft (zum Abtrennen der Gewebeprobe) auf das Gewebe wirkt. Sobald die Sonde mit der Stützeinrichtung an das zu behandelnde Gewebe herangeführt und der Sondenkopf auf das Gewebe aufgesetzt ist, wird der Sondenkopf entsprechend gekühlt, so dass die Gewebeprobe gefroren und an den Sondenkopf angelagert wird. Bei einer Aktivierungszeit von beispielsweise maximal zwei Sekunden der Kühleinrichtung kann vermieden werden, dass die aufgesetzte Stützeinrichtung ebenfalls und unerwünscht anfriert oder sich ein Biopsat ergäbe, das zu groß wäre, um in die Stützeinrichtung aufgenommen werden zu können.

Sonde und Stützeinrichtung können, wie bereits oben erwähnt, gegeneinander verschoben werden. Durch das Verschieben der Elemente gegeneinander wird die Stützeinrichtung gegen das zu behandelnde Gewebe gedrückt. Vorzugsweise ist die Stützeinrichtung derart ausgebildet, dass die Gewebeprobe mit dem Sondenkopf gleichzeitig in das Lumen der Stützeinrichtung eingeholt wird. Die Gewebeprobe wird von dem umliegenden Gewebe abgetrennt (die Probe reißt ab). Dies geschieht sehr viel sanfter als bei herkömmlichen Methoden, da sich die zum Abtrennen der Gewebeprobe erforderliche Zugkraft und die aufgrund der Stützeinrichtung auf das Gewebe wirkende Kraft gegenüber stehen.

Sobald nun die Gewebeprobe von dem umliegenden Gewebe abgetrennt und in die Stützeinrichtung aufgenommen worden ist, kann die Gewebeprobe geborgen werden. Das heißt, die Gewebeprobe wird z. B. mittels des kryochirurgischen Instruments aus dem Arbeitskanal gezogen und ist währenddessen sicher in der Stützeinrichtung untergebracht.

Bevorzugte Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen:
- Fig. 1 Eine Ausführungsform des erfindungsgemäßen kryochirurgischen Instruments, wobei das Instrument teilweise im Schnitt dargestellt ist;
- Fig. 2 einen Ausschnitt des erfindungsgemäßen Instruments, das mit einem Kryochirurgiegerät verbunden ist, mit schematischer Darstellung von Gasleitungseinrichtungen und dem distalen Ende einer Sonde mit Sondenkopf im Schnitt;
- Fig. 3 einen Ausschnitt des erfindungsgemäßen Instruments, mit schematischer Darstellung der Befestigung einer Stützeinrichtung an einer Aufnahmeeinrichtung;
- Fig. 4 das distale Ende der in einem Arbeitskanal eines Endoskops geführten Sonde mit Stützeinrichtung im Schnitt;
- Fig. 5 den Sondenkopf;
- Fig. 6 einen Teil der Gaszuführleitung mit Lochblende, im Schnitt;
- Fig. 7a das distale Ende der Sonde, wobei diese auf ein zu behandelndes Gewebe aufgesetzt ist;
- Fig. 7b das distale Ende der Sonde, wobei eine Gewebeprobe entnommen ist.

In der nachfolgenden Beschreibung werden für gleiche und gleich wirkende Teile.dieselben Bezugsziffern verwendet.

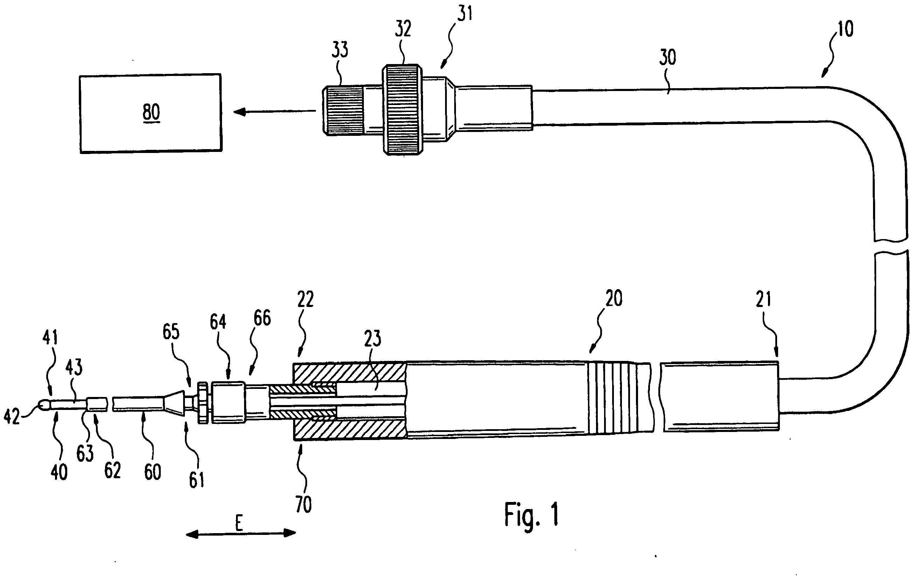

Fig. 1 zeigt eine Ausführungsform des erfindungsgemäßen kryochirurgischen Instruments 10. Mit diesem Instrument zur Biopsie lassen sich Gewebeproben biologischer Gewebe auf einfach Weise gewinnen. Das Instrument 10 ist derart ausgebildet, dass es in der Endoskopie eingesetzt werden kann. Das Instrument weist eine Griffeinrichtung 20 auf, wobei an einem distalen Ende 22 der Griffeinrichtung eine Sonde 40, vorzugsweise zum Einführen in einen Arbeitskanal eines Endoskops (hier nicht gezeigt) vorgesehen ist. Hierzu ist die Sonde 40 starr oder flexibel ausgebildet und kann mit den entsprechenden Endoskopen verwendet werden. Grundsätzlich lässt sich das Instrument 10 auch derart ausbilden, dass es ohne Endoskop, also direkt anwendbar ist.

Die Sonde 40 weist neben einem Sondenkörper 43 an einem distalen Ende 41 einen Sondenkopf 42 auf und ist von einem als eine Stützeinrichtung 60 ausgebildeten Rohr (oder auch Schlauch) ummantelt. Die Stützeinrichtung 60 ist an einer Aufnahmeeinrichtung 64 befestigt, wobei die Aufnahmeeinrichtung 64 in der Griffeinrichtung 20 verschieblich gelagert ist. Die Aufnahmeeinrichtung 64 ist derart an dem distalen Ende 22 der Griffeinrichtung 20 angeordnet, dass ein Bediener die Griffeinrichtung 20 und die Aufnahmeeinrichtung 64 unproblematisch gegeneinander verschieben kann, um letztendlich Sonde 40 und Stützeinrichtung 60 gegeneinander, also relativ zueinander zu verschieben. Die Sonde 40 durchläuft die Aufnahmeeinrichtung 64 und ist in der Griffeinrichtung 20 geführt und dort angekoppelt.

An einem proximalen Ende 21 der Griffeinrichtung 20 ist ein Schlauch 30 angebracht, der wiederum an einem proximalen Ende (im Prinzip das proximale Ende des Instruments) eine Anschlusseinrichtung 31 aufweist. Diese ist in dem gezeigten Zustand durch einen Blindstopfen 33 abgedeckt. Über eine Rändelmutter 32 lässt sich nach dem Entfernen des Blindstopfens 33 das Instrument 10 mit einem kryochirurgischen Gerät 80 und mit einer Gasquelle verbinden. In dem Schlauch 30 sind mindestens eine Gaszuführleitung und mindestens eine Gasabführleitung angeordnet, damit die Sonde 40 einerseits mit Kühlgas bzw. Arbeitsgas versorgt werden kann und andererseits das Gas aus dieser wieder ableitbar ist. Der Schlauch 30 kann auch selbst als Gasabführleitung dienen. Die Gasleitungen durchlaufen den Schlauch 30 und sind über die Griffeinrichtung 20 und die Sonde 40 bis zum Sondenkopf 42 geführt. Hierzu ist eine in der Griffeinrichtung 20 gelagerte (nicht gezeigte) Kupplungseinrichtung vorgesehen, um die Sonde 40 an die aus dem Schlauch 30 kommenden Gasleitungen anzuschließen.

Das kryochirurgische Instrument 10 wird vorzugsweise über das Endoskop an das zu behandelnde Gewebe, aus welchem ein Biopsat gewonnen werden soll, herangeführt und zwar derart, dass der Sondenkopf 42 das Gewebe berührt. Durch das Zuführen des Kühlgases an den Sondenkopf 42 bzw. zumindest in dessen Nähe, lässt sich dieser derart abkühlen, dass das Gewebe bereichsweise an diesen anfriert. Der Sondenkopf 42 ist daher aus thermisch leitfähigem Material, vorzugsweise aus Metall, ausgebildet, um das Anfrieren des Gewebes zu ermöglichen. Zum Kühlen des Sondenkopfes wird der Joule-Thomson-Effekt genutzt, d. h. das Abkühlen eines realen Gases bei gedrosselter Entspannung. Das Gas wird also nur zur Abkühlung des Sondenkopfes benutzt und kommt mit dem Gewebe nicht in Berührung. Das angefrorene Gewebe kann nun von umliegenden Gewebebereichen abgetrennt werden. Die Stützeinrichtung 60 erleichtert diesen Abtrennvorgang. Üblicherweise müsste das an den Sondenkopf 42 angefrorene Gewebe ruckartig von dem übrigen Gewebe abgerissen werden, indem beispielsweise die Sonde 40 ruckartig von dem Gewebe zurückgezogen wird. Beim Ablösen des gefrorenen Präparates, z. B. von der Schleimhaut, bedarf es daher eines großen Kraftaufwandes. Dies ist insbesondere dann problematisch, wenn sich das zu behandelnde Gewebe während des Abtrennvorganges (also bei einer Zugbewegung) mitbewegt. So kann z. B. auf den Dickdarm (wenn von diesem ein Biopsat gewonnen werden soll) keine große Zugkraft ausgeübt werden, da dieser schwimmend im Abdomen liegt.

Um dieser Problematik zu begegnen, weist das erfindungsgemäße kryochirurgische Instrument 10 die oben erwähnte Stützeinrichtung 60 auf. Diese ist so ausgebildet, dass sie als Gegenlager während des Abtrennens des Biopsats von dem umliegenden Gewebe wirkt bzw. nutzbar ist. Das Instrument 10 ist grundsätzlich so ausgebildet, dass das Stützrohr 60 nach dem Anfrieren des Gewebes an den Sondenkopf 42 derart relativ durch das Zusammenwirken von Griffeinrichtung 20 und Aufnahmeeinrichtung 64 zur Sonde 40 in distaler Richtung, also in Richtung des Gewebes bewegbar ist, bis es auf dem Gewebe (das eigentliche Biopsat umgebend) aufliegt bzw. aufsetzt. Durch das gegeneinander Bewegen von Sonde 40 und Stützeinrichtung 60 bzw. Stützrohr lässt sich der Sondenkopf 42 in die Stützeinrichtung 60 aufnehmen, so dass dabei das Biopsat von dem umliegenden Gewebe abgetrennt wird. Das heißt, der Sondenkopf 42 muss derart dimensioniert sein, dass die Stützeinrichtung 60 über diesen geschoben werden kann. Nur so lässt sich ein Abtrennen der Gewebeprobe durch die Zugbewegung am Gewebe bewerkstelligen. Durch das Aufsetzen eines distalen Endes 62 des Stützrohres 60 mit der die Öffnung des Rohres umschließenden Kante (Aufsetzkante) 63 auf das Gewebe wirken die Kraft des Stützrohres (also der Stützeinrichtung) 60 am das Biopsat umgebende Gewebe und gegengleich die Zugkraft zum Abtrennen des gefrorenen Gewebes (Actio gegengleich Reactio). Die Zugkraft am Gewebe zum "Abreißen" der Gewebeprobe wird nun durch die Stützeinrichtung am Gewebe, das die Gewebeprobe umgibt, gemildert. Damit kann das umliegende Gewebe im Wesentlichen in seiner ursprünglichen Position belassen werden und wird nicht ungünstig beansprucht. In jedem Falle sind also Sonde 40 und Stützeinrichtung 60 derart relativ zueinander bewegbar, dass mittels der Stützeinrichtung 60 das umliegende Gewebe, während des Abtrennens der Gewebeprobe mit der Aufnahme der an den Sondenkopf angefrorenen Gewebeprobe in das Stützrohr, abstützbar ist.

Da die Stützeinrichtung 60 gleichzeitig als Einrichtung zum Bergen des Biopsats dient, kann die in die Stützeinrichtung aufgenommene Gewebeprobe geschützt mit der Sonde 40 aus dem Arbeitskanal 90 entfernt werden.

Wie Fig. 1 weiter zeigt, weist die Aufnahmeeinrichtung 64 eine Halteeinrichtung 65 auf, in welcher die Stützeinrichtung 60 mit einem proximalen Ende 61 aufgenommen (z. B. geklemmt oder verschraubt) ist. Die Stützeinrichtung 60 umschließt die Sonde 40 in diesem Ausführungsbeispiel also nur bis zur Aufnahmeeinrichtung 64, während die Sonde 40 in die Griffeinrichtung 20 ohne diese Hülse weiter geführt ist. Ferner weist die Aufnahmeeinrichtung 64 eine Schubeinrichtung 66 auf, mit der die Halteeinrichtung 65 verbunden, z. B. verschraubt oder geklemmt ist. Die Schubeinrichtung 66 ist in der Griffeinrichtung 20 in einem Kanalbereich 23 der Griffeinrichtung 20 geführt, so dass sich Sonde 40 und Stützeinrichtung 60 relativ zueinander in Erstreckungsrichtung E der Sonde bewegen lassen, wie bereits oben beschrieben. Schubeinrichtung 66 und Kanalbereich 23 bilden eine Kopplungseinheit 70 aus. Die Schubeinrichtung 66 stößt beispielsweise mit entsprechenden Bereichen an durch die Griffeinrichtung 20 ausgebildete Anschläge an, um so den Verschiebeweg der Schubeinrichtung 66 zu begrenzen bzw. zu definieren und die Schubeinrichtung 66 in der Griffeinrichtung 20 bzw. in dem Kanalbereich 23 festzuhalten. Auch die Elemente zur Lagerung der Sonde in der Griffeinrichtung können wegbegrenzende Anschläge ausbilden. In diesem Ausführungsbeispiel ist die Schubeinrichtung 66 derart rohrförmig mit einem Kanal ausgebildet, dass die Sonde 40 durch die Schubeinrichtung 66 geführt werden kann.

In diesem Ausführungsbeispiel ist die Schubeinrichtung mit einem der Griffeinrichtung zugewandten Ende in den Kanalbereich der Griffeinrichtung aufgenommen, während das entgegengesetzte Ende der Schubeinrichtung aus der Griffeinrichtung hervorsteht. Somit sind die Griffeinrichtung und die Schubeinrichtung durch einen Bediener fassbar und die beiden Elemente können gegeneinander bewegt, also aufeinander zu oder voneinander weg bewegt werden.

Der proximale Bereich 61 der Stützeinrichtung 60 kann zusätzlich mit einem weiteren Schlauchelement (nicht gezeigt) als Knickschutz umgeben sein, so dass die Sonde mit der Stützeinrichtung im Bereich der Griffeinrichtung äußerst stabil ausgebildet und während des Einsatzes nicht knickbar ist.

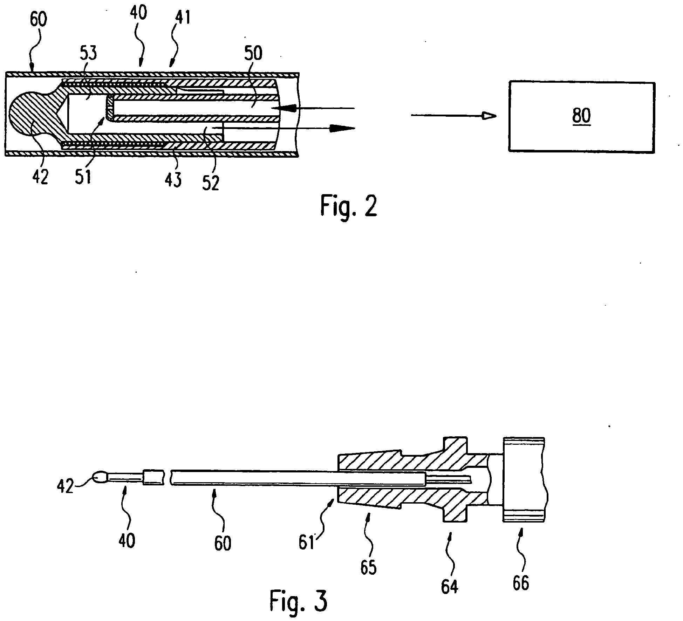

Fig. 2 zeigt einen Ausschnitt des erfindungsgemäßen Instruments, das mit dem Kryochirurgiegerät 80 verbunden ist, wobei schematisch die Gasleitungseinrichtungen 50, 52 auszugsweise und das distale Ende 41 der Sonde 40 mit Sondenkörper 43 und Sondenkopf 42 im Schnitt dargestellt sind. Über die mit dem Kryochirurgiegerät 80 verbundene (nicht gezeigte) Gasquelle wird das Arbeitsgas zum Kühlen des Sondenkopfes 42 durch die Gaszuführleitungen 50 zu dem Sondenkopf 42 geleitet. Das distale Ende 41 der Sonde 40 weist den Sondenkopf 42 auf. An den Sondenkopf 42 heran reichen die Gasleitungen. In diesem Ausführungsbeispiel ist die Gaszuführleitung 50 der Sonde 40 innerhalb der Gasabführleitung 52 angeordnet, wobei die Gasabführleitung 52 hierzu einen größeren Durchmesser aufweist als die Gaszuführleitung 50. Die Gaszuführleitung 50 weist an ihrem sondenkopfnahen Ende eine Lochblende 51 auf, über welche das Gas in einen Hohlraum 53 eintritt, der unmittelbar benachbart des Sondenkopfes 42 ausgebildet ist.

Im Prinzip ist dieser Hohlraum 53 ein sondenkopfnahes Ende der Gasabführleitung 52. Das Gas wird hier durch die Lochblende 51 expandiert und kann dann den vorzugsweise aus Metall (z. B. Edelstahl) ausgebildeten Sondenkopf 42 kühlen. Der Joule-Thomson-Effekt bewirkt durch die Entspannung des Gases eine Kühlung des Sondenkopfs 42. Hierbei wird das unter hohem Druck stehende Gas beim Durchtritt durch eine enge Düse (hier Lochblende) stark abkühlt, so dass die Kryosondenspitze (Sondenkopf) abkühlt und das anliegende Gewebe einfriert. Danach lässt sich das Gas wieder aus dem Hohlraum 53 und damit über die Gasabführleitung 52 aus der Sonde 40 ableiten. Die hier außermittig gelagerte Gaszuführleitung 50 könnte beispielsweise auch koaxial mit der Gasabführleitung 52 angeordnet sein.

Kryochirurgiegeräte sind für verschiedenste Anwendungszwecke vorgesehen, wobei das hier gezeigte beispielsweise nach dem oben erwähnten Joule-Thomson-Effekt arbeitet. Auch mittels flüssigen Stickstoffs können kryochirurgische Eingriffe durchgeführt werden.

Fig. 3 zeigt die Sonde 40 mit Stützeinrichtung 60, wobei das proximale Ende 61 der Stützeinrichtung 60 in der im Schnitt dargestellten Halteeinrichtung 65 der Aufnahmeeinrichtung 64 befestigt ist. Die Stützeinrichtung 60 ist in der Halteeinrichtung 65 derart befestigt, dass die beiden Einrichtungen nicht relativ zueinander bewegbar sind. Allerdings darf die Verklermmung oder Befestigung der Stützeinrichtung 60 in der Aufnahmeeinrichtung 64 die Möglichkeit des gegeneinander Bewegens von Sonde 40 und Stützeinrichtung 60 nicht behindern.

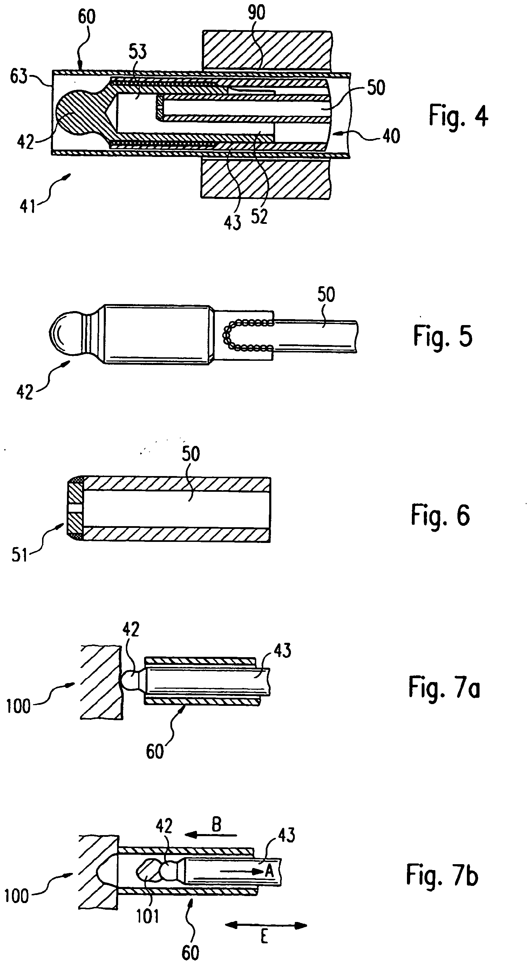

Fig. 4 zeigt das distale Ende 41 der Sonde 40 im Schnitt, wobei die Stützeinrichtung 60 bzw. hier das Stützrohr über den Sondenkopf 42 geschoben ist. Das Stützrohr 60 wird also die Sonde 40 umschließend geführt, so dass mindestens der Sondenkopf 42 in das Stützrohr 40 aufnehmbar und aus diesem wieder freigebbar ist. Ferner ist der Arbeitskanal 90 eines Endoskops gezeigt, in welchen die Sonde 40 mit der Stützeinrichtung 60 eingeführt ist.

Vorzugsweise ist - wie bereits oben erläutert - der Sondenkopf aus Metall ausgebildet. Die Sonde selbst, d. h. der Sondenkörper, ist vorzugsweise aus einem Polyetherketon (PEK), vorzugsweise aus einem Polyetheretherketon (PEEK) oder dergleichen Kunststoff ausgebildet. Auch die Stützeinrichtung ist vorzugsweise aus einem Kunststoff ausgebildet, z. B. aus Perfluor-(Ethylen-Propylen)-Kunststoff (FEP), aus Polytetrafluorethylen (PTFE) oder dergleichen Kunststoff.

Mit Fig. 5 ist der Sondenkopf 42 für sich alleine dargestellt, so wie er dann in den Sondenkörper aufgenommen werden kann. Hier ist er z. B. im Wesentlichen kugelförmig ausgebildet und weist eine z. B. aufgeraute Oberfläche auf. Eine aufgeraute Oberfläche bewirkt eine Oberflächenvergrößerung, so dass das Anlagern (bessere Adhäsion) des Gewebes an den Sondenkopf 42 aufgrund der Struktur unterstützt wird. Damit wird verhindert, dass das Biopsat z. B. beim Bergen verloren geht. Der Sondenkopf kann auch eine Beschichtung aufweisen (die z. B. die Anlagerung des Gewebes erleichtert). Auch eine polierte Ausgestaltung des Sondenkopfes ist natürlich möglich. Die Kugelform vereinfacht die Probengewinnung bei seitlicher Applikation der Kryosonde an dem zu behandelnden Gewebe.

Der Sondenkopf ist hier an einer Art Trägerelement integral mit diesem vorgesehen, um über das Trägerelement die Sonde zu positionieren und den Sondenkopf so zu befestigen. Das Trägerelement weist an einem Ende ein Langloch auf, an welchem die Gaszuführleitung 50 zur Zugentlastung befestigt, z. B. angeschweißt ist. Die Gaszuführleitung wird also z. B. durch Laserschweißen umlaufen am Langloch befestigt.

Fig. 6 zeigt die Lochblende 51, die an dem sondenkopfnahen Ende der Gaszuführleitung ausgebildet ist. Bei Durchtritt durch den Lochbereich findet eine Entspannung des Gases derart statt, dass es gekühlt wird und damit eine Kühlung des Sondenkopfes 42 erfolgt.

Die Fig. 7a und 7b zeigen die Entnahme der Gewebeprobe 101 von dem zu behandelnden Gewebe 100. Hier sind lediglich Sondenende mit Sondenkopf 42 und Stützrohr 60 dargestellt. Das Endoskop ist nicht gezeigt. In Fig. 7a steht der Sondenkopf aus dem Stützrohr (der Stützeinrichtung) hervor und ist an dem zu behandelnden Gewebe 100 aufgesetzt. Sobald das Gewebe bereichsweise an dem Sondenkopf angefroren ist, kann dieser Bereich, der schließlich die Gewebeprobe 101 bildet, durch das gegeneinander Bewegen von Sonde 40 und Stützeinrichtung 60 (über Griffeinrichtung und Schubeinrichtung) von dem umliegenden Gewebe 100 abgetrennt und in das Stützrohr 60 eingeholt werden - wie Fig. 7b zeigt -, während gleichzeitig das Stützrohr 60 auf dem Gewebe 100, das die Gewebeprobe 101 umgibt bzw. umgab, aufsetzt und entgegen der Zugkraft durch die Sonde 40 zurückhält. Die Pfeile deuten etwaige Bewegungsrichtungen A, B von Sonde 40 und/oder Stützrohr 60 in Erstreckungsrichtung E der Sonde an.

Es sei abschließend bemerkt, dass es ein wesentlicher Punkt der Erfindung ist, bei dem kryochirurgischen Instrument eine Einrichtung vorzusehen, die ein Gewebe, aus welchem eine Gewebeprobe entnommen werden soll, derart abstützt, dass eine für die Entnahme der Gewebeprobe erforderliche Zugkraft an dem umliegenden Gewebe abgemildert wird. Dies ist insbesondere dann vorteilhaft, wenn das zu behandelnde Gewebe beweglich im Körper des Patienten aufgehängt ist und/oder derart elastisch ist, dass es die Zugbewegung mitverfolgen würde.

### Bezugszeichenliste

- 10: Kryochirurgisches Instrument
- 20: Griffeinrichtung
- 21: Proximales Ende der Griffeinrichtung
- 22: Distales Ende der Griffeinrichtung
- 23: Kanalbereich
- 30: Schlauch
- 31: Anschlusseinrichtung
- 32: Rändelmutter
- 33: Blindstopfen
- 40: Sonde
- 41: Distales Ende der Sonde
- 42: Sondenkopf
- 43: Sondenkörper
- 50: Gaszuführleitung
- 51: Lochblende
- 52: Gasabführleitung

- 53: Hohlraum
- 60: Stützeinrichtung
- 61: Proximales Ende der Stützeinrichtung
- 62: Distales Ende der Stützeinrichtung
- 63: Aufsetzkante
- 64: Aufnahmeeinrichtung
- 65: Halteeinrichtung
- 66: Schubeinrichtung
- 70: Kopplungseinheit
- 80: Kryochirurgisches Gerät, Kryochirurgiegerät
- 90: Arbeitskanal eines Endoskop
- 100: Zu behandelndes Gewebe, umliegendes Gewebe
- 101: Gewebeprobe
- E: Erstreckungsrichtung Sonde
- A: Eine mögliche Bewegungsrichtung Sonde
- B: Eine mögliche Bewegungsrichtung Stützeinrichtung

## Patentansprüche

1. Kryochirurgisches Instrument, umfassend
- eine Sonde (40) zum Heranführen eines Sondenkopfes (42) an ein zu behandelndes biologisches Gewebe,
- Gasleitungseinrichtungen (50, 52) zum Zuführen von Kühlgas von einer Gasquelle eines kryochirurgischen Gerätes (80) an den Sondenkopf (42) und zum Abführen des Kühlgases von diesem,
wobei der Sondenkopf (42) derart ausgebildet ist, dass mittels des zugeführten Gases ein begrenzter Gewebebereich zur Gewinnung einer Gewebeprobe (101) kühlbar ist,
, wobei
eine Stützeinrichtung (60) vorgesehen ist, in der die Sonde (40) geführt und die relativ zu der Sonde (40) derart bewegbar und auf das umliegende Gewebe (100) aufsetzbar ist, dass sie eine Gegenkraft auf das umliegende Gewebe (100) ausübt, während die Sonde eine Zugkraft auf die Gewebeprobe (101) ausübt, um diese abzutrennen, so dass mittels der Stützeinrichtung (60) das umliegende Gewebe (100) während des Abtrennens der Gewebeprobe (101) abgestützt wird, **dadurch gekennzeichnet, dass** die Gewebeprobe (101) vom umliegenden Gewebe (100) in einem an dem Sondenkopf (42) angefrorenen Zustand abtrennbar ist und die Stützeinrichtung (60) derart ausgebildet ist, dass die an den Sondenkopf (42) angefrorene Gewebeprobe (101) zu deren Bergung mit dem Sondenkopf (42) in die Stützeinrichtung (60) aufnehmbar ist.

2. Kryochirurgisches Instrument nach Anspruch 1,
**dadurch gekennzeichnet, dass**
die Sonde (40) einen starren oder flexiblen Schaft oder Katheter aufweist und durch einen Instrumentenkanal (90) eines starren oder flexiblen Endoskops an das zu behandelnde Gewebe heranführbar ist.

3. Kryochirurgisches Instrument nach Anspruch 1 oder 2,
**dadurch gekennzeichnet, dass**
eine Griffeinrichtung (20) zur Handhabung der Sonde (40) vorgesehen ist, wobei ein proximales Ende der Sonde (40) in der Griffeinrichtung (20) befestigt ist.

4. Kryochirurgisches Instrument nach einem der vorhergehende Ansprüche,
**dadurch gekennzeichnet, dass**
die Gasleitungseinrichtungen eine durch die Sonde (40) verlaufende Gaszuführleitung (50) aufweisen, so dass ein Hohlraum (53) im Inneren der Sonde (40) zum Kühlen des Sondenkopfes (42) mit Gas befüllbar ist.

5. Kryochirurgisches Instrument nach einem der vorhergehende Ansprüche,
**dadurch gekennzeichnet, dass**
die Gasleitungseinrichtungen eine durch die Sonde (40) verlaufende Gasabführleitung (52) aufweisen, wobei die Gasabführleitung (52) derart ausgebildet ist, dass sie den Hohlraum (53) umfasst.

6. Kryochirurgisches Instrument nach einem der vorhergehende Ansprüche,
**dadurch gekennzeichnet, dass**
die Stützeinrichtung (60) derart ausgebildet ist, dass sie die Sonde (40) rohr- oder schlauchförmig umschließt.

7. Kryochirurgisches Instrument nach einem der vorhergehende Ansprüche, insbesondere nach einem der Ansprüche 3 bis 6,
**dadurch gekennzeichnet, dass**
eine Aufnahmeeinrichtung (64) zur Aufnahme eines proximalen Endes (61) der Stützeinrichtung (60) vorgesehen ist, wobei die Aufnahmeeinrichtung (64) und die Griffeinrichtung (20) verschieblich zueinander mittels einer Kopplungseinheit (70) miteinander verbunden sind.

8. Kryochirurgisches Instrument nach einem der vorhergehende Ansprüche, insbesondere nach Anspruch 7,
**dadurch gekennzeichnet, dass**
die Kopplungseinheit (70) derart ausgebildet ist, dass eine Schubeinrichtung (66) der Aufnahmeeinrichtung (64) und ein Kanalbereich (23) der Griffeinrichtung (20) ineinander greifen, so dass die Stützeinrichtung (60) und die Sonde (40) mittels einer Schub- oder Zugbewegung von Griffeinrichtung (20) und/oder Aufnahmeeinrichtung (64) gegeneinander längs einer Erstreckungsrichtung (E) der Sonde über eine definierte Weglänge derart bewegbar sind, dass mindestens der Sondenkopf (42) in die Stützeinrichtung (60) aufnehmbar oder von dieser freigebbar ist.

9. Kryochirurgisches Instrument nach einem der vorhergehende Ansprüche,
**dadurch gekennzeichnet, dass**
eine Öffnung der Stützeinrichtung (60) umschließende Aufsetzkante (63) an einem distalen Ende (62) der Stützeinrichtung (60) zur Kontaktierung des zu behandelnden Gewebes abgestumpft ausgebildet ist.

## Claims

1. Cryosurgical instrument, comprising:
- a probe (40) for guiding a probe head (42) to biological tissue to be treated,
- gas line apparatuses (50, 52) for supplying coolant gas from a gas source of a cryosurgical piece of equipment (80) to the probe head (42) and for venting the coolant gas from the latter, the probe head (42) being designed such that the supplied gas can be used to cool a delimited tissue region for obtaining a tissue sample (101),
provision being made for a support apparatus (60), in which the probe (40) is guided and which can be moved relative to the probe (40) and placed onto the surrounding tissue (100) such that it exerts an opposing force onto the surrounding tissue (100) while the probe exerts a pulling force on the tissue sample (101) in order to sever the latter, such that the surrounding tissue (100) is supported by means of the support apparatus (60) while the tissue sample (101) is being severed, **characterized in that** the tissue sample (101) can be severed from the surrounding tissue (100) in a state where it is frozen onto the probe head (42) and the support apparatus (60) is designed such that the tissue sample (101) frozen onto the probe head (42) can be held in the support apparatus (60) together with the probe head (42) for the purpose of retrieving said tissue sample (101).

2. Cryosurgical instrument according to Claim 1,
**characterized in that**
the probe (40) has a rigid or flexible shaft or catheter and can be guided to the tissue to be treated through an instrument channel (90) of a rigid or flexible endoscope.

3. Cryosurgical instrument according to Claim 1 or 2,
**characterized in that**
provision is made for a grip apparatus (20) for handling the probe (40), with a proximal end of the probe (40) being attached in the grip apparatus (20).

4. Cryosurgical instrument according to one of the preceding claims,
**characterized in that**
the gas line apparatuses have a gas supply line (50) running through the probe (40) such that a cavity (53) in the interior of the probe (40) can be filled with gas for cooling the probe head (42).

5. Cryosurgical instrument according to one of the preceding claims,
**characterized in that**
the gas line apparatuses have a gas venting line (52) running through the probe (40), the gas venting line (52) being designed such that it comprises the cavity (53).

6. Cryosurgical instrument according to one of the preceding claims,
**characterized in that**
the support apparatus (60) is designed such that it encloses the probe (40) in a pipe-like or tubelike fashion.

7. Cryosurgical instrument according to one of the preceding claims, more particularly according to one of Claims 3 to 6,
**characterized in that**
provision is made for a holding apparatus (64) for holding a proximal end (61) of the support apparatus (60), the holding apparatus (64) and the grip apparatus (20) being interconnected such that they can be displaced with respect to one another by means of a coupling unit (70).

8. Cryosurgical instrument according to one of the preceding claims,
more particularly according to Claim 7,
**characterized in that**
the coupling unit (70) is designed such that a translatory apparatus (66) of the holding apparatus (64) and a channel region (23) of the grip apparatus (20) grip into one another, such that the support apparatus (60) and the probe (40) can be moved against one another over a defined path length along a direction of extent (E) of the probe by means of a pushing or pulling motion of grip apparatus (20) and/or holding apparatus (64) such that at least the probe head (42) can be held by the support apparatus (60) or be released by the latter.

9. Cryosurgical instrument according to one of the preceding claims,
**characterized in that**
a placement edge (63), which surrounds an opening of the support apparatus (60), is formed, with a blunt design, at a distal end (62) of the support apparatus (60) for contacting the tissue to be treated.

## Revendications

1. Instrument cryochirurgical, comprenant
- une sonde (40) pour amener une tête de sonde (42) sur un tissu biologique à traiter,
- des dispositifs de conduites de gaz (50, 52) pour amener du gaz de refroidissement d'une source de gaz d'un appareil cryochirurgical (80) à la tête de sonde (42) et pour évacuer le gaz de refroidissement de celle-ci,
dans lequel la tête de sonde (42) est réalisée de telle manière qu'une zone limitée du tissu puisse être refroidie au moyen du gaz amené pour le prélèvement d'un échantillon de tissu (101),
dans lequel il est prévu un dispositif de support (60), dans lequel la sonde (40) est guidée et qui peut être déplacé par rapport à la sonde (40) et appliqué sur le tissu environnant (100), de telle manière qu'il exerce une force opposée sur le tissu environnant (100), tandis que la sonde exerce une force de traction sur l'échantillon de tissu (101) afin de séparer celui-ci, de telle manière que le tissu environnant (100) soit supporté au moyen du dispositif de support (60) pendant la séparation de l'échantillon de tissu (101),
**caractérisé en ce que** l'échantillon de tissu (101) peut être séparé du tissu environnant (100) dans un état congelé sur la tête de sonde (42) et le dispositif de support (60) est réalisé de telle manière que l'échantillon de tissu (101) congelé sur la tête de sonde (42) puisse être logé dans le dispositif de support (60) pour sa récupération avec la tête de sonde (42).

2. Instrument cryochirurgical selon la revendication 1, **caractérisé en ce que** la sonde (40) présente un guide ou un cathéter rigide ou flexible et peut être amenée sur le tissu à traiter à travers un canal d'instrument (90) d'un endoscope rigide ou flexible.

3. Instrument cryochirurgical selon la revendication 1 ou 2, **caractérisé en ce qu'**il est prévu un dispositif de saisie (20) pour la manipulation de la sonde (40), dans lequel une extrémité proximale de la sonde (40) est fixée dans le dispositif de saisie (20).

4. Instrument cryochirurgical selon l'une quelconque des revendications précédentes, **caractérisé en ce que** les dispositifs de conduites de gaz présentent une conduite d'arrivée de gaz (50) s'étendant à travers la sonde (40), de telle manière qu'un espace creux (53) à l'intérieur de la sonde (40) puisse être rempli de gaz pour le refroidissement de la tête de sonde (42).

5. Instrument cryochirurgical selon l'une quelconque des revendications précédentes, **caractérisé en ce que** les dispositifs de conduites de gaz présentent une conduite d'évacuation de gaz (52) s'étendant à travers la sonde (40), dans lequel la conduite d'évacuation de gaz (52) est réalisée de telle manière qu'elle entoure l'espace creux (53).

6. Instrument cryochirurgical selon l'une quelconque des revendications précédentes, **caractérisé en ce que** le dispositif de support (60) est réalisé de telle manière qu'il entoure la sonde (40) sous la forme d'un tube ou d'un tuyau.

7. Instrument cryochirurgical selon l'une quelconque des revendications précédentes, en particulier selon l'une quelconque des revendications 3 à 6, **caractérisé en ce qu'**il est prévu un dispositif de réception (64) destiné à recevoir une extrémité proximale (61) du dispositif de support (60), dans lequel le dispositif de réception (64) et le dispositif de saisie (20) sont assemblés l'un à l'autre de façon coulissante l'un par rapport à l'autre au moyen d'une unité de couplage (70).

8. Instrument cryochirurgical selon l'une quelconque des revendications précédentes, en particulier selon la revendication 7, **caractérisé en ce que** l'unité de couplage (70) est réalisée de telle manière qu'un dispositif de poussée (66) du dispositif de réception (64) et une région de canal (23) du dispositif de saisie (20) s'engagent l'un dans l'autre, de telle manière que le dispositif de support (60) et la sonde (40) puissent être déplacés l'un par rapport à l'autre sur une longueur de course définie le long d'une direction d'extension (E) de la sonde, au moyen d'un mouvement de poussée ou de traction du dispositif de saisie (20) et/ou du dispositif de réception (64), de telle manière qu'au moins la tête de sonde (42) puisse être logée dans le dispositif de support (60) ou puisse être libérée de celui-ci.

9. Instrument cryochirurgical selon l'une quelconque des revendications précédentes, **caractérisé en ce qu'**une face d'application (63) entourant une ouverture du dispositif de support (60) est formée de façon tronquée sur une extrémité distale (62) du dispositif de support (60) pour le contact avec le tissu à traiter.
